# EUROPÄISCHE PATENTSCHRIFT

(11) **EP 1 296 685 B1**
(45) Veröffentlichungstag und Bekanntmachung des Hinweises auf die Patenterteilung: **28.02.2007**
(21) Anmeldenummer: 01949412.9
(22) Anmeldetag: 13.06.2001
(51) Int. Cl.: A61K 31/495, A61K 9/24

(54) **RETARD-ZUBEREITUNGEN VON CHINOLON-ANTIBIOTIKA UND VERFAHREN ZU IHRER HERSTELLUNG**
SUSTAINED-RELEASE PREPARATIONS OF QUINOLONE ANTIBIOTICS AND METHOD FOR PREPARATION THEREOF
PREPARATIONS A LIBERATION PROLONGEE A BASE D'ANTIBIOTIQUES DE TYPE QUINOLONES ET PROCEDE D'ELABORATION DESDITES PREPARATIONS

(30) Priorität: 26.06.2000 DE 10031043
(43) Veröffentlichungstag der Anmeldung: 02.04.2003
(73) Patentinhaber: Bayer HealthCare AG, 51368 Leverkusen (DE)
(72) Erfinder: KANIKANTI, Venkata-Rangarao, 51381 Leverkusen (DE); RUPP, Roland, 51467 Bergisch Gladbach (DE); WEBER, Wolfgang, 51065 Köln (DE); DEURINGER, Peter, 51061 Köln (DE); HENCK, Jan-Olav, 47877 Willich (DE); STASS, Heino, 51061 Köln (DE); NISHIOKA, Takaaki, Nabari-shi, Mie 518-0614 (JP); KATAKAWA, Yoshifumi, Kusatsu-shi, Shiga 525-0025 (JP); TANIGUCHI, Chika, Nishinomiya-shi, Hyogo 662-0831 (JP); ICHIHASHI, Hitoshi, Suita-shi, Osaka 564-0042 (JP)
(74) Vertreter: Linkenheil, Dieter
(86) Internationale Anmeldenummer: PCT/EP2001/006695
(87) Internationale Veröffentlichungsnummer: WO 2002/000219

(56) Entgegenhaltungen:
- WO-A-99/15172
- US-A- 4 443 428
- US-A- 5 286 754
- US-B1- 6 261 601

## Beschreibung

Die vorliegende Erfindung betrifft feste, oral applizierbare Matrix-Zubereitungen von Chinolon-Antibiotika mit verzögerter Freisetzung und ein Verfahren zu ihrer Herstellung.

Wirkstoffe aus der Klasse der Chinolone werden seit langer Zeit als Breitband-Antibiotika eingesetzt, und es sind zahlreiche Darreichungsformen auf dem Markt erhältlich, wie z.B. Tabletten, Infusionslösungen, Augentropfen etc.

Für viele Arzneimittel - so auch für die Klasse der Chinolone - sind Formulierungen wünschenswert, die nach einmal täglicher Verabreichung eine kontrollierte, lang anhaltende und gleichmäßige Freisetzung des Wirkstoffs gewährleisten. Auf diese Weise können die gewünschte Wirkstoff-Konzentration im Plasma (nachfolgend: "Plasmaspiegel") und die therapeutische Wirkung über einen längeren Zeitraum ohne große Schwankungen aufrechterhalten werden. Formulierungen, die den Wirkstoff auf diese Weise über einen längeren Zeitraum freisetzen, werden als Retard- oder controlled-release(CR)-Zubereitungen bezeichnet.

Es ist jedoch sehr schwierig, oral verabreichbare Chinolon-Zubereitungen zu entwickeln, die trotz einer nur einmal täglichen Verabreichung eine genügend hohe antibiotische Wirkung garantieren; der Patient muss daher täglich mindestens zwei Dosen einnehmen. Es ist aber wünschenswert, die Einnahmefrequenz bei solchen Chinolon-Antibiotika auf einmal täglich zu reduzieren.

Zur Herstellung von Zubereitungen mit kontrollierter Wirkstoff-Freisetzung sind im Prinzip verschiedene Techniken bekannt. So wird oft angestrebt, die Zubereitung für einen längeren Zeitraum im Magen zu belassen, um die Resorption des zu retardierenden Wirkstoffs im Resorptionsfenster (d.h. in dem Abschnitt des Gastrointestinaltrakts, in dem die Absorption stattfindet), schnell und vollständig zu ermöglichen. Die Verweilzeit im Magen hängt jedoch stark von Art und Nährwert der im Magen befindlichen Nahrung ab (S. S. Davis in G. Hardy et al., Drug Delivery to the Gastrointestinal Tract, Ellis Holwood Ltd., Chichester, England 1989). Um die Verweilzeit im Magen zu verlängern, wurden verschiedene Ansätze erprobt, die entweder
a) die Dichte der Zubereitung erhöhen (EP-A 265 061),
b) spezielle Zusätze wie Ammoniummyristat verwenden, die bekanntlich den Weitertransport von Zubereitungen in den Gastrointestinaltrakt verlangsamen (R. Gröning; G. Heung, Int. J. Pharm. 56, 111 (1989)),
c) im Magen quellende Zubereitungen (Ballontablette) einsetzen (Agyilirah et al., Int. J. Pharm. 75, 241 (1991)),
d) Zubereitungen mit großer räumlicher Ausdehnung einsetzen (EP-A 235 718) oder
e) bioadhäsive Zubereitungen einsetzen, die vorzugsweise an den Schleimhäuten des Gastrointestinaltrakts haften sollen (R. Khosla, S. S. Davis, J. Pharm. Pharmacol. 39, 47 (1987)).

Eine andere Retard-Technik bedient sich einer Matrix aus hydrophilen Polymeren und gegebenenfalls pharmazeutischen Hilfsstoffen, in die der Wirkstoff eingebettet ist. In wäßriger Umgebung quillt das Polymer zu einem Gel, das dann entweder (zusammen mit dem schwer löslichen Wirkstoff) langsam erodiert oder durch das der (gut lösliche) Wirkstoff diffundiert. Das Polymer kann hydrophil, hydrophob oder gemischt hydrophil/hydrophob sein. Mittlerweile sind Matrixtabletten sehr beliebt, weil sie vergleichsweise preiswert und gut verträglich sind und auf herkömmlichen Anlagen hergestellt werden können.

Eine andere Methode besteht in der Verwendung gepufferter bzw. pH-sensibler Umhüllungen, die eine kontrollierte Freisetzung in bestimmten Abschnitten des Gastrointestinaltrakts erlauben.

Eine technisch aufwendige Methode besteht in der Verwendung osmotischer Systeme (OROS), die nach folgendem Prinzip funktionieren: Durch eine wasserdurchlässige Membran dringt Wasser langsam in die Tablette und führt dort zum Anquellen eines wasserquellbaren Inhaltsstoffs; der durch die Volumenvergrößerung entstehende Druck treibt den Wirkstoff durch eine für diesen Zweck vorgesehene Öffnung aus der Tablette.

All diese Techniken weisen Nachteile auf, insbesondere teure und komplizierte Herstellungsmethoden, inter- und intraindividuelle Variabilität oder Abhängigkeit der gewünschten Wirkung von der Körperhaltung.

Bei der Herstellung von Retard-Zubereitungen ist auch jeweils darauf zu achten, wo die Resorption des Wirkstoffs stattfinden kann: Je kleiner das Resorptionsfenster, desto schwieriger gestaltet sich die Herstellung von Retard-Zubereitungen. Chinolone wie Ciprofloxacin beispielsweise werden vorwiegend im oberen Teil des Dünndarms (Zwölffingerdarm) resorbiert; die Resorption im unteren Teil des Dünndarms und im Dickdarm ist signifikant geringer (S. Harder et al., Br. J. clin. Pharmacol. 30, 35-39, (1990)). Daher muss der Wirkstoff, um maximale Bioverfügbarkeit zu erreichen, freigesetzt sein, bevor die Zubereitung dieses Resorptionsfenster verläßt. Außerdem ist der starke Einfluß des pH-Werts des umgebenden Mediums auf die Löslichkeit von Chinolon-Wirkstoffen zu berücksichtigen; sie nimmt mit steigendem pH-Wert ab.

Aufgabe der Erfindung war es daher, einfache Retard-Zubereitungen von Chinolon-Antibiotika zur Verfügung zu stellen, die bei einmal täglicher Verabreichung eine ausreichende therapeutische Wirkung gewährleisten.

Gegenstand der Erfindung ist daher eine oral applizierbare. Zubereitung enthaltend ein Chinolon-Antibiotikum, dadurch gekennzeichnet, dass sie ein Gemisch von
a) wasserquellbarem Polymer und
b) einer Mischung von mindestens zwei Derivaten des Chinolon-Antibiotikums
enthält.

Diese oral applizierbare antibiotische Matrix-Zubereitung enthaltend Chinolon-Wirkstoff ist auch dadurch gekennzeichnet, dass sie 80 % des Wirkstoffs sowohl in 0,1 N Salzsäure als auch in Acetatpuffer bei p_{H} 4,5 im USP XXIV Paddle-Test bei 50 Umdrehungen pro Minute/37°C innerhalb von 1 bis 4 Stunden freisetzt. Um das Aufschwimmen der Tablette beim Test zu verhindern, kann sie in einem Drahtkäfig platziert werden, wie er z.B. in der japanischen Pharmakopoeia beschrieben ist.

Der Begriff "Chinolon-Wirkstoffe" bezeichnet im Rahmen der vorliegenden Erfindung die Klasse der als Antiinfektiva verwendbaren Stoffe mit einem Chinolon-Grundgerüst, insbesondere der Chinoloncarbonsäuren. Bevorzugte Chinolon-Wirkstoffe umfassen Ciprofloxacin, Olamufloxacin, Clinafloxacin, Trovafloxacin, Cadrofloxacin, Alatrofloxacinmesylate, Gatifloxacin, Rufloxacin, Sparfloxacin, Levofloxacin, Irloxacin, Grepafloxacin, Moxifloxacin, Prulifloxacin, Pazufloxacin, Gemifloxacin, Sitafloxacin, Tosulfloxacin, Amifloxacin, Lomefloxacin, R-Lomefloxacin und Nitrosoxacin-A. Der bevorzugteste Chinolon-Wirkstoff ist Ciprofloxacin und seine Hydrate.

Der Begriff "Chinolon-Wirkstoffe" umfaßt im Rahmen der vorliegenden Erfindung auch Chinolonderivate, die erst im Körper den aktiven Wirkstoff freisetzen (sog. Prodrugs), z.B. Ester einer Chinoloncarbonsäure.

Nach einer bevorzugten Ausführungsform enthält die erfindungsgemäße Zubereitung als Wirkstoff eine Kombination, vorzugsweise eine Mischung, zweier verschiedener Chinolonderivate. Beispiel einer solchen erfindungsgemäßen Ausführungsform wäre eine Zubereitung, die als Wirkstoff eine Mischung zweier verschiedener Chinolonsalze enthält.

Eine bevorzugte Ausführungsform betrifft Zubereitungen, die als Wirkstoff die Mischung einer freien Chinolonbase und ihres Salzes enthält. Besonders bevorzugt sind Mischungen von Ciprofloxacin-Hydrochlorid und Ciprofloxacin-Betain.

Ciprofloxacin-Hydrochlorid ist beispielsweise bei niedrigen p_{H}-Werten gut löslich; die Löslichkeit ist aber beim p_{H}-Wert des Intestinaltraktes (≥ 6,5) signifikant vermindert. Es hat sich jedoch herausgestellt, dass Mischungen von Ciprofloxacin-Hydrochlorid und freier Ciprofloxacin-Base (Betain) in einem Gewichtsverhältnis von 1:20 bis 20:1, insbesondere 1:10 bis zu 10:1 weitgehend pH-unabhängig (im pH-Bereich von 1 bis 4,5) aus der Zubereitung freigesetzt werden. Ein gleichwirkender Effekt kann auch dadurch erzielt werden, dass Mischungen anderer Derivate, z.B. Salze, Basen oder Prodrugs des Wirkstoffes verwendet werden. Mischungen von Stereoisomeren fallen im Rahmen der Erfindung hingegen nicht unter den Begriff "Kombination zweier verschiedener Chinolonderivate", wohl aber Mischungen von Hydrat und Anhydrat.

Eine besondere Ausführungsform der erfindungsgemäßen Zubereitungen betrifft Matrixtabletten. Bevorzugte Matrixtabletten enthalten einen Retardteil (CR-Teil) und einen schnell freisetzenden Teil (IR-Teil). Als retardierende Polymere für die Matrix eignen sich wasserquellbare Polymere, z.B. Polysaccharide wie Stärken und Stärkederivate (Mais-, Weizen-, Reis-, Kartoffelstärke, Carboxymethylstärken, Natriumstärkeglykolate), Celluloseether wie Alkylcellulosen, Hydroxyalkylcellulosen, Carboxyalkylcellulosen und deren Alkalimetallsalze (Methyl-, Hydroxymethyl-, Hydroxyethyl-, Hydroxypropyl-, Natriumcarboxymethylcellulosen, vernetzte Carboxymethyleellulosen), Dextrine, Dextran, Pektine, Polyosen, Gummi arabicum, Tragant, Carrageen, Galactomannane wie Guar-Gummi, Algin, Alginsäure und Alginate, Polypeptide und Proteine wie Gelatine und Casein, weiterhin Chitinderivate wie Chitosan, vollsynthetische Polymere wie (Meth-)Acrylsäure-Copolymere (Methylmethacrylat-, Hydroxymethylmethacrylat-Copolymere), Polyvinylalkohol, unvernetztes Polyvinylpyrrolidon und Vinylpyrrolidon-Copolymere, und Mischungen der genannten Verbindungen. Da die wasserquellbaren Polymeren in Gegenwart von Wasser Gele bilden, kann man sie auch als "gelbildende Polymere" bezeichnen.

Häufig werden für Retardzubereitungen hochviskose Polymere verwendet. Bei der vorliegenden Erfindung wurde jedoch festgestellt, dass niedrigviskose Polymere das Freisetzungsverhalten der Zubereitungen positiv beeinflussen. Prinzipiell können zum Zweck der Retardierung alle hydrophilen Polymeren niedriger Viskosität benutzt werden. Der Begriff "niedrigviskos" im Rahmen der vorliegenden Erfindung bedeutet eine (scheinbare) Viskosität von 5 bis 400 mPa • s (cP), vorzugsweise von maximal 75 cP, insbesondere von maximal 50 cP gemessen mit einem Rotationsviskosimeter als 2 gew.-%-ige wässrige Lösung bei 20°C.

Besonders bevorzugt ist Hydroxypropylmethylcellulose (HPMC). Insbesondere bevorzugt ist HPMC der USP XXIV-Spezifikation 2910, d.h. mit einem Methoxyanteil von 28 bis 30 Gew.-% und einem Hydroxypropoxyanteil von 7 bis 12 Gew.-%, z.B. Metolose^{®} 60 SH (Shinetsu, Japan). Der gewünschte Retardierungsgrad der Zubereitung kann durch Wahl von Viskosität und Menge der HPMC eingestellt werden.

Bevorzugte HPMC besitzt eine Viskosität von 5 bis 400 cP, vorzugsweise von maximal 75 cP, insbesondere von maximal 50 cP (jeweils gemessen mit einem Rotationsviskosimeter als 2 gew.-%-ige wässrige Lösung bei 20°C).

Der Anteil des hydrophilen Polymers, vorzugsweise der HPMC, kann innerhalb weiter Grenzen variiert werden. Vorzugsweise wird jedoch 1 Gewichtsteil hydrophiles Polymer pro 2 bis 20, vorzugsweise pro 5 bis 15, Gewichtsteile Wirkstoff eingesetzt.

Um die Freisetzung des Wirkstoffes aus der Dosisform auch im Dünndarm zu gewährleisten und den p_{H}-Wert der äußeren Schicht und der Umgebung der Zubereitung im sauren Bereich zu halten und dadurch das Risiko der Ausfällung des Wirkstoffes im höherem p_{H}-Wert der Intestinalflüssigkeit möglichst weitgehend zu verhindern, kann eine organische Säure in die Zubereitung (falls vorhanden, vorzugsweise in den Retardteil) eingearbeitet werden; auf diese Weise wird der Wirkstoff in einer für die Resorption zugänglicheren Form bereitgestellt. Zu diesem Zweck bevorzugte organische Säuren besitzen 2 bis 10 C-Atome und 1 bis 4 Carboxylgruppen, wie beispielsweise Essigsäure, Malonsäure, Bernsteinsäure, Fumarsäure, Weinsäure und Zitronensäure.

Neben Wirkstoff, hydrophilem retardierendem Polymer und gegebenenfalls organischer Säure können die erfindungsgemäßen Zubereitungen auch Sprengmittel, z.B. vemetztes Polyvinylpyrrolidon wie ^{®}Kollidon CL, Gleitmittel, z.B. kolloidales Siliciumdioxid wie ^{®}Aerosil, hydrierte pflanzliche Öle, Stearinsäure, Talkum oder deren Mischungen, Schmiermittel, z.B. Magnesiumstearat, sowie gegebenenfalls andere Hilfsstoffe enthalten. Sowohl Gleitmittel als auch Schmiermittel werden vorzugsweise vor der Tablettierphase in das Granulat eingearbeitet.

Die Tabletten können anschließend lackiert werden, um gegebenenfalls einen bitteren Geschmack des Wirkstoffes zu maskieren, den Wirkstoff vor Lichteinfluss zu schützen und/oder um die Tablette ästhetisch ansprechender zu machen. Die Lackierung kann z.B. durch Aufsprühen einer wäßrigen Suspension aus: Filmbildner, z.B. HPMC, Weichmacher, z.B. Polyethylenglykol, und lichtstreuende und lichtabsorbierende Pigmente, z.B. Titandioxid, erfolgen. Zum Abtrocknen des Wassers kann während der Lackierung heiße Luft auf das Tablettenbett gerichtet werden.

Mit den beschriebenen Komponenten lassen sich Retardzubereitungen herstellen. Neben dem Retardteil (CR-Teil) kann auch ein schnell freisetzender Anteil (IR-Teil) eingesetzt werden, um eine rasche Anflutung und einen höheren Plasmaspiegel zu erhalten. Unter schnell freisetzenden (IR-)Zubereitungen werden im Rahmen der vorliegenden Erfindung solche verstanden, die den Wirkstoff gemäß USP XXIV-Paddle-Methode beliebig schnell, vorzugsweise innerhalb von 3 Minuten bis weniger als 60 Minuten freisetzen. Die schnelle Freisetzung kann durch Variation der Zusammensetzung, z.B. durch Variation des Sprengmittelanteils, oder durch die Herstellungsparameter innerhalb bestimmter Grenzen kontrolliert werden. Schnell freisetzende Teile der erfindungsgemäßen Zubereitungen müssen nicht unbedingt zwei verschiedene Chinolonderivate enthalten.

Es ist also möglich, Kombinationspräparate herzustellen, die in einer Single-Unit-Dosisform Zubereitungen mit verschiedenen Freisetzungsprofilen enthalten: So können Zubereitungen mit unterschiedlichem Freisetzungsprofil verwendet werden, um den Plasmaspiegel zeitlich genau zu steuern. Unter "Kombinationspräparaten" im Sinne der Erfindung werden nicht nur Single-Unit-Dosisformen (sog. Fixkombinationen) und Kombinationspackungen, die voneinander getrennt je eine Zubereitung mit unterschiedlichem Freisetzungsprofil (kit-of-parts) enthalten, verstanden, sondern auch gleichzeitig oder zeitlich versetzt applizierte IR- bzw. CR-Teile, sofern sie zur Behandlung oder Prophylaxe derselben Krankheit eingesetzt werden.

Gegenstand der vorliegenden Erfindung ist also auch eine Kombinationszubereitung, die einen schnell freisetzenden Teil und einen Retardteil aufweist, z.B. in Form einer Zweischichttablette.

Der schnell freisetzende Teil kann Chinolon-Wirkstoff (z.B. Ciprofloxacin-Hydrochlorid und Ciprofloxacin-Betain), Sprengmittel (z.B. vernetztes Polyvinylpyrrolidon wie Kollidon® CL), Gleitmittel (kolloidales Siliciumdioxid, z.B. Aerosil^{®}) und Schmiermittel (z.B. Magnesiumstearat) sowie gegebenenfalls organische Säure oder andere Hilfsstoffe enthalten. Der Retardanteil kann Wirkstoff (Ciprofloxacin-Hydrochlorid und Ciprofloxacin-Betain), das retardierende Polymer (z.B. HPMC geringer Viskosität), organische Säure (z.B. Bernsteinsäure), ein Gleitmittel (z.B. kolloidales Siliciumdioxid) und ein Schmiermittel (z.B. Magnesiumstearat) und gegebenenfalls weitere Hilfsstoffe enthalten. Die Ausgangsmaterialien für den schnell freisetzenden und den Retardteil können (z.B. mit Nass- oder Trockengranulationstechniken) vor der Tablettierung granuliert werden. Das Granulat kann mit Gleit- und Schmiermittel gemischt werden, und das komprimierfähige (ready-to-compress) Granulat der beiden Schichten kann (z.B. unter Anwendung herkömmlicher Zweischicht-Tablettiermaschinen) zu Zweischichttabletten tablettiert werden. Ein Teil des Gleitmittels könnte auch granuliert werden.

Da die Zugabe einer organischen Säure die Freisetzungsrate des Wirkstoffes, insbesondere von Ciprofloxacin-Hydrochlorid und -Betain, steigert, kann es sich auch empfehlen, dem IR-Teil organische Säure beizumischen.

Die erfindungsgemäßen Retard-Zubereitungen enthalten zweckmäßigerweise 500 bis 1000 mg Wirkstoff, berechnet als Betain, pro Single-Unit-Dosisform. Unter "Single-Unit-Dosisformen" werden solche Zubereitungen verstanden, die als Einzelgabe verabreicht werden, z.B. Tabletten, Dragees oder Kapseln.

Zur Herstellung von erfindungsgemäßen Retard-Zubereitungen mit IR- und CR-Teil kann beispielsweise folgendes Verfahren angewandt werden: Für die Herstellung des IR-Teils wird der Wirkstoff (vorzugsweise als Mischung zweier Derivate) mit Sprengmittel, insbesondere Kollidon CL, vermischt und granuliert und mit Gleitmittel, insbesondere Aerosil, und Schmiermittel, insbesondere Magnesiumstearat, gemischt, um zunächst kompaktierfähiges (ready-to-compress) IR-Granulat zu erhalten.
Für den Retardteil wird der Wirkstoff (als Mischung zweier Derivate) mit Säure, z.B. Bernsteinsäure, und gelbildendem Polymer, insbesondere HPMC, vermischt und granuliert. Dieses CR-Granulat wird mit Gleitmittel, insbesondere Aerosil®, und Schmiermittel, insbesondere Magnesiumstearat, gemischt, um komprimierfähiges (ready-to-compress) CR-Granulat zu erhalten. Das (ready-to-compress) CR-Granulat und das IR-Granulat werden mit einer herkömmlichen Zweischicht-Tablettiermaschine zu einer Zweischichttablette tablettiert. Die erhaltene Tablette kann dann lackiert werden.

Die folgenden Ausführungsbeispiele sollen den Erfindungsgegenstand anhand von Zweischichttabletten erläutern.

### Beispiele

### Beispiel 1

| **Menge in mg** | **Einsatzstoffe** |
|---|---|
| 366,70 | Ciprofloxacin-Hydrochlorid |
| 41,70 | Ciprofloxacin-Betain |
| 46,700 | Kollidon CL** |
| 4,30 | Aerosil 200*** |
| 4,70 | Magnesiumstearat |
| 464,10 | *Subtotal IR-Teil* |
| 302,70 | Ciprofloxacin-Hydrochlorid |
| 464,30 | Ciprofloxacin-Betain |
| 125,40 | Bernsteinsäure |
| 103,10 | Hydroxypropylmethylcellulose 50 cP* |
| 5,20 | Aerosil 200*** |
| 9,30 | Magnesiumstearat |
| 1010,00 | *Subtotal CR-Teil* |
| 18,00 | Hydroxypropylmethylcellulose 15 cP* |
| 6,00 | Titandioxid |
| 6,00 | Polyethylenglykol 400 **** |
| 30,00 | *Lack sub-total* |
| 23 x 9,5 mm | Oblongtablette |

| | |
|---|---|
| * Viskosität, jeweils gemessen als 2 gew.-%-ige wäßrige Lösung bei 20°C ** vernetztes Polyvinylpyrrolidon *** kolloidales Siliciumdioxid, spezifische Oberfläche 200 m²/g **** Die Zahlenangabe bezieht sich auf das mittlere Molekulargewicht | |

### Beispiel 2

| **Menge in mg** | **Einsatzstoffe** |
|---|---|
| 183,40 | Ciprofloxacin-Hydrochlorid |
| 20,90 | Ciprofloxacin-Betain |
| 22,30 | Kollidon CL |
| 2,30 | Magnesiumstearat |
| 1,10 | Aerosil 200 |
| 230,00 | *Subtotal IR-Teil* |
| 151,40 | Ciprofloxacin-Hydrochlorid |
| 232,10 | Ciprofloxacin-Betain |
| 64,00 | Bernsteinsäure |
| 52,30 | Hydroxypropylmethylcellulose 15 cP |
| 7,60 | Magnesiumstearat |
| 2,60 | Aerosil 200 |
| 510,00 | *Subtotal CR-Teil* |
| 12,00 | Hydroxypropylmethylcellulose 15 cP |
| 4,00 | Polyethylenglykol 400 |
| 4,00 | Titandioxid |
| 20,00 | *Lack sub-total* |
| 19 x 8 mm | Oblongtablette |

### Beispiel 3

| **Menge in mg** | **Einsatzstoffe** |
|---|---|
| 183,40 | Ciprofloxacin-Hydrochlorid |
| 20,90 | Ciprofloxacin-Betain |
| 22,30 | Kollidon CL |
| 2,30 | Magnesiumstearat |
| 1,10 | Aerosil 200 |
| 230,00 | *Subtotal IR-Teil* |
| 151,40 | Ciprofloxacin-Hydrochlorid |
| 232,10 | Ciprofloxacin-Betain |
| 65,10 | Bernsteinsäure |
| 73,00 | Hydroxypropylmethylcellulose 15 cP |
| 10,70 | Magnesiumstearat |
| 2,70 | Aerosil 200 |
| 535,00 | *Subtotal CR-Teil* |
| 12,00 | Hydroxypropylmethylcellulose 15 cP |
| 4,00 | Polyethylenglykol 3350 |
| 4,00 | Titandioxid |
| 20,00 | *Lack sub-total* |
| 19 x 8 mm | Oblongtablette |

### Beispiel 4

| **Menge in mg** | **Einsatzstoffe** |
|---|---|
| 183,40 | Ciprofloxacin-Hydrochlorid |
| 20,90 | Ciprofloxacin-Betain |
| 22,30 | Kollidon CL |
| 2,30 | Magnesiumstearat |
| 1,10 | Aerosil 200 |
| 230,00 | *Subtotal IR-Teil* |
| 151,40 | Ciprofloxacin-Hydrochlorid |
| 232,10 | Ciprofloxacin-Betain |
| 64,00 | Bernsteinsäure |
| 72,00 | Hydroxypropylmethylcellulose 50 cP |
| 7,90 | Magnesiumstearat |
| 2,60 | Aerosil 200 |
| 530,00 | *Subtotal CR-Teil* |
| 12,00 | Hydroxypropylmethylcellulose 15 cP |
| 4,00 | Polyethylenglykol 400 |
| 4,00 | Titandioxid |
| 20,00 | *Lack sub-total* |
| 19 x 8 mm | Oblongtablette |

### Beispiel 5

| **Menge in mg** | **Einsatzstoffe** |
|---|---|
| 262,00 | Ciprofloxacin-Hydrochlorid |
| 29,80 | Ciprofloxacin-Betain |
| 8,90 | Bernsteinsäure |
| 42,20 | Kollidon CL |
| 1,80 | Aerosil 200 |
| 5,30 | Magnesiumstearat |
| 350,00 | *Subtotal IR-Teil* |
| 116,40 | Ciprofloxacin-Hydrochlorid |
| 178,50 | Ciprofloxacin-Betain |
| 134,00 | Bernsteinsäure |
| 87,80 | Hydroxypropylmethylcellulose 15 cP |
| 2,70 | Aerosil 200 |
| 10,60 | Magnesiumstearat |
| 530,00 | *Subtotal CR-Teil* |
| 12,00 | Hydroxypropylmethylcellulose 15 cP |
| 4,00 | Polyethylenglykol 400 |
| 4,00 | Titandioxid |
| 20,00 | *Lack sub-total* |
| 19 x 8 mm | Oblongtablette |

### Beispiel 6

| **Menge in mg** | **Einsatzstoffe** |
|---|---|
| 183,40 | Ciprofloxacin-Hydrochlorid |
| 20,90 | Ciprofloxacin-Betain |
| 6,20 | Bernsteinsäure |
| 24,70 | Kollidon CL |
| 1,20 | Aerosil 200 |
| 3,60 | Magnesiumstearat |
| 240,00 | *Subtotal IR-Teil* |
| 151,40 | Ciprofloxacin-Hydrochlorid |
| 232,10 | Ciprofloxacin-Betain |
| 174,00 | Bernsteinsäure |
| 95,70 | Hydroxypropylmethylcellulose 15 cP |
| 3,40 | Aerosil 200 |
| 13,40 | Magnesiumstearat |
| 670,00 | *Subtotal CR-Teil* |
| 12,00 | Hydroxypropylmethylcellulose 15 cP |
| 4,00 | Polyethylenglykol 400 |
| 4,00 | Titandioxid |
| 20,00 | *Lack sub-total* |
| 19 x 8 mm | Oblongtablette |

### Beispiel 7

| **Menge in mg** | **Einsatzstoffe** |
|---|---|
| 366,70 | Ciprofloxacin-Hydrochlorid |
| 41,70 | Ciprofloxacin-Betain |
| 46,60 | Kollidon CL |
| 4,70 | Magnesiumstearat |
| 2,30 | Aerosil 200 |
| 462,00 | *Subtotal IR-Teil* |
| 302,70 | Ciprofloxacin-Hydrochlorid |
| 464,30 | Ciprofloxacin-Betain |
| 125,30 | Bernsteinsäure |
| 103,00 | Hydroxypropylmethylcellulose 15 cP |
| 20,50 | Magnesiumstearat |
| 5,20 | Aerosil 200 |
| 1021,00 | *Subtotal CR-Teil* |
| 18,00 | Hydroxypropylmethylcellulose 15 cP |
| 6,00 | Polyethylenglykol 3350 |
| 6,00 | Titandioxid |
| 30,00 | *Lack Subtotal* |
| 23 x 9,5 mm | Oblongtablette |

### Vergleichsbeispiel A

| **Menge in mg** | **Einsatzstoffe** |
|---|---|
| 357,00 | Ciprofloxacin-Betain |
| 58,00 | Kollidon CL |
| 6,00 | Magnesiumstearat |
| 4,00 | Aerosil 200 |
| 425,00 | *Subtotal IR-Teil* |
| 833,00 | Ciprofloxacin-Betain |
| 108,00 | Bernsteinsäure |
| 108,00 | Hydroxypropylmethylcellulose 15cP |
| 16,00 | Magnesiumstearat |
| 10,00 | Aerosil 200 |
| 1075,00 | *Subtotal CR-Teil* |
| 18,00 | Hydroxypropylmethylcellulose 15 cP |
| 6,00 | Polyethylenglykol 400 |
| 6,00 | Titandioxid |
| 30,00 | *Lack Subtotal* |
| 23 x 9,5 mm | Oblongtablette |

### Vergleichsbeispiel B

| **Menge in mg** | **Einsatzstoffe** |
|---|---|
| 357,00 | Ciprofloxacin-Betain |
| 58,00 | Kollidon CL |
| 6,00 | Magnesiumstearat |
| 4,00 | Aerosil 200 |
| 425,00 | *Subtotal IR-Teil* |
| 833,00 | Ciprofloxacin-Betain |
| 108,00 | Bernsteinsäure |
| 108,00 | Hydroxypropylmethylcellulose 3 cP |
| 16,00 | Magnesiumstearat |
| 10,00 | Aerosil 200 |
| 1075,00 | *Subtotal CR-Teil* |
| 18,00 | Hydroxypropylmethylcellulose 15 cP |
| 6,00 | Polyethylenglykol 400 |
| 6,00 | Titandioxid |
| 30,00 | *Lack Subtotal* |
| 23 x 9,5 mm | Oblongtablette |

Die erfindungsgemäßen Zubereitungen der Beispiele 1 bis 7 zeigen in 0,1 N HCl-Lösung oder Acetatpuffer bei pH 4,5 in herkömmlichen Freisetzungsapparaturen (USP-Paddle-Test) weitgehend pH-unabhängige Freisetzung, während die Zubereitungen der Vergleichsbeispiele A und B starke pH-Abhängigkeit zeigen.

## Patentansprüche

1. Oral applizierbare Zubereitung enthaltend ein Chinolon-Antibiotikum, **dadurch gekennzeichnet, dass** sie ein Gemisch von
a) wasserquellbarem Polymer und
b) einer Mischung von mindestens zwei Derivaten des Chinolon-Antibiotikums
enthält.

2. Zubereitung nach Anspruch 1, **dadurch gekennzeichnet, dass** das Polymer eine Viskosität von 5 bis 400 × 10⁻³ Pa.s (5 bis 400 cP), gemessen als 2 gew.-%-ige wässrige Lösung bei 20°C, hat.

3. Zubereitung nach Anspruch 1 oder 2, **dadurch gekennzeichnet, dass** sie zusätzlich einen Hilfsstoff ausgewählt aus der Gruppe umfassend organische Säure, Sprengmittel, Gleitmittel und Schmiermittel enthält.

4. Zubereitung nach einem der vorstehenden Ansprüche, **dadurch gekennzeichnet, dass** sie ein Kombinationspräparat, enthaltend einen schnell freisetzenden Teil (IR-Teil) und einen Retardteil (CR-Teil), ist.

5. Zubereitung nach dem vorstehenden Anspruch, **dadurch gekennzeichnet, dass** der IR-Teil Chinolon-Antibiotikum, Sprengmittel, Gleitmittel und Schmiermittel enthält.

6. Zubereitung nach einem der beiden vorstehenden Ansprüche, **dadurch gekennzeichnet, dass** der CR-Teil Chinolon-Antibiotikum, Polymer, organische Säure, Gleitmittel und Schmiermittel enthält.

7. Zubereitung nach Anspruch 4, **dadurch gekennzeichnet, dass** der IR-Teil Chinolon-Antibiotikum, Sprengmittel, Gleitmittel und Schmiermittel enthält und der CR-Teil Chinolon-Antibiotikum, Polymer, organische Säure, Gleitmittel und Schmiermittel enthält.

8. Zubereitung nach einem der Ansprüche 4 bis 7, **dadurch gekennzeichnet, dass** der IR-Teil und der CR-Teil jeweils eine Mischung von zwei Derivaten des Chinolon-Antibiotikums enthalten.

9. Zubereitung nach einem der Ansprüche 4 bis 8, **dadurch gekennzeichnet, dass** der IR-Teil zusätzlich organische Säure enthält.

10. Zubereitung nach einem der Ansprüche 4 bis 9 in der Form einer Zweischichttablette.

11. Zubereitung nach einem der vorstehenden Ansprüche, **dadurch gekennzeichnet, dass** sie als Mischung zweier Derivate des Chinolon-Antibiotikums eine Mischung eines Salzes mit der freien Base enthält.

12. Zubereitung nach einem der vorstehenden Ansprüche, **dadurch gekennzeichnet, dass** sie als Mischung zweier Derivate des Chinolon-Antibiotikums eine Mischung zweier Salze enthält.

13. Zubereitung nach einem der vorstehenden Ansprüche, **dadurch gekennzeichnet, dass** das Chinolon-Antibiotikum Ciprofloxacin ist.

14. Zubereitung nach einem der vorstehenden Ansprüche, **dadurch gekennzeichnet, dass** die zwei Derivate Ciprofloxacin-Hydrochlorid und Ciprofloxacin-Betain sind.

15. Zubereitung nach dem vorstehenden Anspruch, **dadurch gekennzeichnet, dass** Ciprofloxacin-Hydrochlorid und Ciprofloxacin-Betain in einem Gewichtsverhältnis von 1:20 bis 20:1 enthalten sind.

16. Zubereitung nach einem der vorstehenden Ansprüche, **dadurch gekennzeichnet, dass** das Polymer ausgewählt ist aus der Gruppe, umfassend Polysaccharide, Celluloseether und deren Alkalimetallsalze, Dextrine, Dextran, Pektine, Polyosen, Gummi arabicum, Tragant, Carrageen, Galactomannane, Algin, Alginsäure, Alginate, Polypeptide, Proteine, Chitinderivate, vollsynthetische Polymere und deren Mischungen, enthält.

17. Zubereitung nach dem vorstehenden Anspruch, **dadurch gekennzeichnet, dass** das Polymer Hydroxypropylmethylcellulose ist.

18. Zubereitung nach einem der vorstehenden Ansprüche, **dadurch gekennzeichnet, dass** sie eine organische Säure mit 2 bis 10 C-Atomen und 1 bis 4 Carboxylgruppen enthält.

19. Zubereitung nach dem vorstehenden Anspruch, **dadurch gekennzeichnet, dass** die organische Säure ausgewählt ist aus der Gruppe, umfassend Essigsäure, Malonsäure, Bernsteinsäure, Fumarsäure, Weinsäure und Zitronensäure.

20. Zubereitung nach einem der vorstehenden Ansprüche, **dadurch gekennzeichnet, dass** sie vernetztes Polyvinylpyrrolidon als Sprengmittel enthält.

21. Zubereitung nach einem der vorstehenden Ansprüche, **dadurch gekennzeichnet, dass** sie ein Gleitmittel ausgewählt aus der Gruppe, umfassend kolloidales Siliciumdioxid, hydrierte pflanzliche Öle, Stearinsäure, Talkum und deren Mischungen, enthält.

22. Zubereitung nach einem der vorstehenden Ansprüche, **dadurch gekennzeichnet, dass** sie Magnesiumstearat als Schmiermittel enthält.

23. Zubereitung nach einem der vorstehenden Ansprüche, **dadurch gekennzeichnet, dass** das Polymer eine Viskosität von maximal 75 × 10⁻³ Pa.s (75 cP), gemessen als 2 gew.-%-ige wässrige Lösung bei 20°C, hat.

24. Zubereitung nach einem der vorstehenden Ansprüche, **dadurch gekennzeichnet, dass** das Polymer Hydroxypropylmethylcellulose einer Viskosität von maximal 75 × 10⁻³Pa.s (75 cP), gemessen als 2 gew.-%-ige wässrige Lösung bei 20°C, ist.

25. Zubereitung nach dem vorstehenden Anspruch , **dadurch gekennzeichnet, dass** die Hydroxypropylmethylcellulose eine Viskosität von maximal 50 × 10⁻³ Pa.s (50 cP), gemessen als 2 gew.-%-ige wässrige Lösung bei 20°C, aufweist.

26. Zubereitung nach einem der vorstehenden Ansprüche, **dadurch gekennzeichnet, dass** sie pro Gewichtsteil Polymer 2 bis 20 Gewichtsteile Wirkstoffmischung enthält.

27. Zubereitung nach einem der vorstehenden Ansprüche, **dadurch gekennzeichnet, dass** sie Hydroxypropylmethylcellulose als Polymer und pro Gewichtsteil Hydroxypropylmethylcellulose 2 bis 20 Gewichtsteile Wirkstoffmischung enthält.

28. Zubereitung nach einem der vorstehenden Ansprüche, **dadurch gekennzeichnet, dass** sie 500 bis 1000 mg Chinolon-Antibiotikum, berechnet als Betain, pro Single-Unit-Dosisform enthält.

29. Verfahren zur Herstellung einer Zubereitung nach einem der vorstehenden Ansprüche, wonach
ein Teil des Wirkstoffs mit Sprengmittel vermischt, granuliert und mit Gleit- und Schmiermittel gemischt wird (IR-Teil), und
ein anderer Teil des Wirkstoffs mit organischer Säure und Polymer vermischt, granuliert und mit Gleit- und Schmiermittel gemischt wird (CR-Teil),
und IR- und CR-Teil zu Kombinationstabletten tablettiert und gegebenenfalls die resultierenden Tabletten lackiert werden.

30. Verfahren nach dem vorstehenden Anspruch, **dadurch gekennzeichnet, dass**
ein Teil des Wirkstoffs mit Sprengmittel vermischt, granuliert und mit Gleit- und Schmiermittel gemischt wird (IR-Teil), und
ein anderer Teil des Wirkstoffs mit Säure und Hydroxypropylmethylcellulose vermischt, granuliert und mit Gleit- und Schmiermittel gemischt wird (CR-Teil),
und IR- und CR-Teil zu Kombinationstabletten tablettiert und die resultierenden Tabletten lackiert werden.

31. Verfahren nach einem der Ansprüche 29 oder 30, **dadurch gekennzeichnet, dass** der IR-Teil und der CR-Teil zu einer Zweischichttablette tablettiert werden.

32. Verfahren nach einem der Ansprüche 29 bis 31, **dadurch gekennzeichnet, dass** dem IR-Teil organische Säure beigemischt wird.

33. Zubereitung nach einem der vorstehenden Ansprüche, **dadurch gekennzeichnet, dass** sie 80 % des Wirkstoffs sowohl in 0,1 N Salzsäure als auch in Acetatpuffer bei p_{H} 4,5 im USP XXIV Paddle-Test bei 50 Umdrehungen pro Minute/37°C innerhalb von 1 bis 4 Stunden freisetzt.

## Claims

1. Orally administrable preparation comprising a quinolone antibiotic, **characterized in that** it contains a mixture of
a) water-swellable polymer and
b) a mixture of at least two derivatives of the quinolone antibiotic.

2. Preparation according to Claim 1, **characterized in that** the polymer has a viscosity of 5 to 400 × 10⁻³ Pa.s (5 to 400 cP), measured as a 2% strength by weight aqueous solution at 20°C.

3. Preparation according to Claim 1 or 2, **characterized in that** it additionally contains an adjuvant selected from the group comprising organic acid, disintegrant, glidant and lubricant.

4. Preparation according to any one of the preceding claims, **characterized in that** it is a combination preparation comprising an instant release (IR) part and a controlled release (CR) part.

5. Preparation according to the preceding claim, **characterized in that** the IR part contains quinolone antibiotic, disintegrant, glidant and lubricant.

6. Preparation according to either of the two preceding claims, **characterized in that** the CR part contains quinolone antibiotic, polymer, organic acid, glidant and lubricant.

7. Preparation according to Claim 4, **characterized in that** the IR part contains quinolone antibiotic, disintegrant, glidant and lubricant and the CR part contains quinolone antibiotic, polymer, organic acid, glidant and lubricant.

8. Preparation according to any one of Claims 4 to 7, **characterized in that** the IR part and the CR part each contain a mixture of two derivatives of the quinolone antibiotic.

9. Preparation according to any one of Claims 4 to 8, **characterized in that** the IR part additionally contains organic acid.

10. Preparation according to any one of Claims 4 to 9 in the form of a two-layer tablet.

11. Preparation according to any one of the preceding claims, **characterized in that** it contains, as mixture of two derivatives of the quinolone antibiotic, a mixture of a salt with the free base.

12. Preparation according to any one of the preceding claims, **characterized in that** it contains, as mixture of two derivatives of the quinolone antibiotic, a mixture of two salts.

13. Preparation according to any one of the preceding claims, **characterized in that** the quinolone antibiotic is ciprofloxacin.

14. Preparation according to any one of the preceding claims, **characterized in that** the two derivatives are ciprofloxacin hydrochloride and ciprofloxacin betaine.

15. Preparation according to the preceding claim, **characterized in that** ciprofloxacin hydrochloride and ciprofloxacin betaine are included in a weight ratio of 1:20 to 20:1.

16. Preparation according to any one of the preceding claims, **characterized in that** the polymer is selected from the group comprising polysaccharides, cellulose ethers and their alkali metal salts, dextrins, dextran, pectins, polyoses, gum arabic, tragacanth, carrageenan, galactomannans, algin, alginic acid, alginates, polypeptides, proteins, chitin derivatives, fully synthetic polymers and mixtures thereof.

17. Preparation according to the preceding claim, **characterized in that** the polymer is hydroxypropylmethylcellulose.

18. Preparation according to any one of the preceding claims, **characterized in that** it contains an organic acid having 2 to 10 carbon atoms and 1 to 4 carboxyl groups.

19. Preparation according to the preceding claim, **characterized in that** the organic acid is selected from the group comprising acetic acid, malonic acid, succinic acid, fumaric acid, tartaric acid and citric acid.

20. Preparation according to any one of the preceding claims, **characterized in that** it contains crosslinked polyvinylpyrrolidone as disintegrant.

21. Preparation according to any one of the preceding claims, **characterized in that** it contains a glidant selected from the group comprising colloidal silica, hydrogenated vegetable oils, stearic acid, talc and mixtures thereof.

22. Preparation according to any one of the preceding claims, **characterized in that** it contains magnesium stearate as lubricant.

23. Preparation according to any one of the preceding claims, **characterized in that** the polymer has a viscosity of at most 75 × 10⁻³ Pa.s (75 cP), measured as a 2% strength by weight aqueous solution at 20°C.

24. Preparation according to any one of the preceding claims, **characterized in that** the polymer is hydroxypropylmethylcellulose of a viscosity of at most 75 × 10⁻³ Pa.s (75 cP), measured as a 2% strength by weight aqueous solution at 20°C.

25. Preparation according to the preceding claim, **characterized in that** the hydroxypropylmethylcellulose has a viscosity of at most 50 × 10⁻³ Pa.s (50 cP), measured as a 2% strength by weight aqueous solution at 20°C.

26. Preparation according to any one of the preceding claims, **characterized in that** it contains 2 to 20 parts by weight of active compound mixture per part by weight of polymer.

27. Preparation according to any one of the preceding claims, **characterized in that** it contains hydroxypropylmethylcellulose as polymer and 2 to 20 parts by weight of active compound mixture per part by weight of hydroxypropylmethylcellulose.

28. Preparation according to any one of the preceding claims, **characterized in that** it contains 500 to 1000 mg of quinolone antibiotic, reckoned as betain, per single unit dose form.

29. Process for the production of a preparation according to any one of the preceding claims,
according to which
one part of the active compound is mixed with disintegrant, granulated and mixed with glidant and lubricant (IR part), and
another part of the active compound is mixed with organic acid and polymer, granulated and mixed with lubricant and glidant (CR part),
and IR part and CR part are tabletted to give combination tablets and optionally the resulting tablets are coated.

30. Process according to the preceding claim, **characterized in that**
one part of the active compound is mixed with disintegrant, granulated and mixed with glidant and lubricant (IR part), and
another part of the active compound is mixed with acid and hydroxypropylmethylcellulose, granulated and mixed with lubricant and glidant (CR part),
and IR part and CR part are tabletted to give combination tablets and the resulting tablets are coated.

31. Process according to either of Claims 29 and 30, **characterized in that** the IR part and the CR part are tabletted to give a two-layer tablet.

32. Process according to any one of Claims 29 to 31, **characterized in that** organic acid is admixed to the IR part.

33. Preparation according to any one of the preceding claims, **characterized in that** it releases 80% of the active compound both in 0.1 N hydrochloric acid and in acetate buffer at pH 4.5 in the USP XXIV paddle test at 50 revolutions per minute/37°C in the course of 1 to 4 hours.

## Revendications

1. Préparation à usage oral contenant un antibiotique de type quinolone, **caractérisée en ce qu'**elle contient un mélange
a) d'un polymère gonflable dans l'eau et
b) d'un mélange d'au moins deux dérivés de l'antibiotique de type quinolone.

2. Préparation selon la revendication 1, **caractérisée en ce que** le polymère possède une viscosité de 5 à 400 x 10⁻³ Pa.s (5 à 400 cP) à 20 °C.

3. Préparation selon la revendication 1 ou 2, **caractérisée en ce qu'**elle contient en plus un adjuvant sélectionné parmi le groupe comprenant des acides organiques, des agents désagrégeants, des agents antifriction et des lubrifiants.

4. Préparation selon une des revendications précédentes, **caractérisée en ce qu'**il s'agit d'une préparation d'association contenant une partie libérée rapidement (partie IR) et une partie retard (partie CR).

5. Préparation selon la revendication précédente, **caractérisée en ce que** la partie IR contient l'antibiotique de type quinolone, l'agent désagrégeant, l'agent antifriction et le lubrifiant.

6. Préparation selon une des revendications précédentes, **caractérisée en ce que** la partie CR contient l'antibiotique de type quinolone, le polymère, l'acide organique, l'agent antifriction et le lubrifiant.

7. Préparation selon la revendication 4, **caractérisée en ce que** la partie IR contient l'antibiotique de type quinolone, l'agent désagrégeant, l'agent antifriction et le lubrifiant et la partie CR contient l'antibiotique de type quinolone, le polymère, l'acide organique, l'agent antifriction et le lubrifiant.

8. Préparation selon une des revendications 4 à 7, **caractérisée en ce que** la partie IR et la partie CR contiennent chacune un mélange de deux dérivés de l'antibiotique de type quinolone.

9. Préparation selon une des revendications 4 à 8, **caractérisée en ce que** la partie IR contient en plus l'acide organique.

10. Préparation selon une des revendications 4 à 9 sous la forme d'un comprimé bicouche.

11. Préparation selon une des revendications précédentes, **caractérisée en ce qu'**elle contient comme mélange des deux dérivés de l'antibiotique de type quinolone, un mélange d'un sel avec la base libre.

12. Préparation selon une des revendications précédentes, **caractérisée en ce qu'**elle contient comme mélange des deux dérivés de l'antibiotique de type quinolone, un mélange de deux sels.

13. Préparation selon une des revendications précédentes, **caractérisée en ce que** l'antibiotique de type quinolone est la ciprofloxacine.

14. Préparation selon une des revendications précédentes, **caractérisée en ce que** les deux dérivés sont le chlorhydrate de ciprofloxacine et la ciprofloxacine-bétaine.

15. Préparation selon la revendication précédente, **caractérisée en ce que** le chlorhydrate de ciprofloxacine et la ciprofloxacine-bétaine sont contenus dans un rapport pondéral compris entre 1:20 et 20:1.

16. Préparation selon une des revendications précédentes, **caractérisée en ce que** le polymère est sélectionné parmi le groupe comprenant les composés suivants: polysaccharides, éthers de cellulose et sels de métaux alcalins de ceux-ci, dextrines, dextrane, pectines, polyoses, gomme arabique, gomme adragante, carragaheen, galactomannane, algine, acide alginique, alginates, polypeptides, protéines, dérivés de chitine, polymères entièrement synthétiques et dérivés de ceux-ci.

17. Préparation selon la revendication précédente, **caractérisée en ce que** le polymère est l'hydroxypropylméthylcellulose.

18. Préparation selon une des revendications précédentes, **caractérisée en ce qu'**elle contient un acide organique comportant 2 à 10 atomes de C et 1 à 4 groupes carboxyle.

19. Préparation selon la revendication précédente, **caractérisée en ce que** l'acide organique est sélectionné parmi le groupe comprenant l'acide acétique, l'acide malonique, l'acide succinique, l'acide fumarique, l'acide tartrique et l'acide citrique.

20. Préparation selon une des revendications précédentes, **caractérisée en ce qu'**elle contient de la polyvinylpyrrolidone réticulée comme agent désagrégeant.

21. Préparation selon une des revendications précédentes, **caractérisée en ce qu'**elle contient un agent antifriction sélectionné parmi le groupe comprenant le dioxyde de silicium colloïdal, les huiles végétales hydrogénées, l'acide stéarique, le talc et les mélanges de ceux-ci.

22. Préparation selon une des revendications précédentes, **caractérisée en ce qu'**elle contient du stéarate de magnésium comme lubrifiant.

23. Préparation selon une des revendications précédentes, **caractérisée en ce que** le polymère possède une viscosité de 75 x 10⁻³ Pa.s (75 cP) au maximum, déterminée dans une solution aqueuse à 2 % en poids à 20 °C.

24. Préparation selon une des revendications précédentes, **caractérisée en ce que** le polymère est une hydroxypropylméthycellulose d'une viscosité de 75 x 10⁻³ Pa.s (75 cP) au maximum, déterminée dans une solution aqueuse à 2 % en poids à 20 °C.

25. Préparation selon la revendication précédente, **caractérisée en ce que** l'hydroxypropylméthycellulose présente une viscosité de 50 x 10⁻³ Pa.s (50 cP) au maximum, déterminée dans une solution aqueuse à 2 % en poids à 20 °C.

26. Préparation selon une des revendications précédentes, **caractérisée en ce qu'**elle contient par partie en poids du polymère 2 à 20 parties en poids du mélange de substances actives.

27. Préparation selon une des revendications précédentes, **caractérisée en ce qu'**elle contient de l'hydroxypropylméthylcellulose comme polymère et par partie en poids d'hydroxypropylméthylcellulose 2 à 20 parties en poids du mélange de substances actives.

28. Préparation selon une des revendications précédentes, **caractérisée en ce qu'**elle contient 500 à 1000 mg d'antibiotique de type quinolone, calculé comme bétaïne, par forme à dose unique.

29. Procédé de production d'une préparation selon une des revendications précédentes,
selon lequel
une partie de la substance active est mélangée avec l'agent désagrégeant, granulée et mélangée avec l'agent antifriction et le lubrifiant (partie IR) et
une autre partie de la substance active est mélangée avec l'acide organique et le polymère, granulée et mélangée avec l'agent antifriction et le lubrifiant (partie CR), et les parties IR et CR sont transformées en comprimés d'association et les comprimés obtenus sont laqués le cas échéant.

30. Procédé selon la revendication précédente, **caractérisé en ce qu'**une partie de la substance active est mélangée avec l'agent désagrégeant, granulée et mélangée avec l'agent antifriction et le lubrifiant (partie IR) et
une autre partie de la substance active est mélangée avec l'acide et l'hydroxypropylméthylcellulose, granulée et mélangée avec l'agent antifriction et le lubrifiant (partie CR), et
les parties IR et CR sont transformées en comprimés d'association et les comprimés obtenus sont laqués le cas échéant.

31. Procédé selon une des revendications 29 à 30, **caractérisé en ce que** la partie IR et la partie CR sont transformées en un comprimé bicouche.

32. Procédé selon une des revendications 29 à 31, **caractérisé en ce que** l'acide organique est additionné à la partie IR.

33. Préparation selon une des revendications précédentes, **caractérisées en ce qu'**elle libère en 1 à 4 heures 80 % de la substance active aussi bien dans de l'acide chlorhydrique 0,1 N que dans un tampon acétate à pH 4,5 dans le Paddle-test USP XXIV à 50 tours/minute et à 37 °C.
